# NEW EUROPEAN PATENT SPECIFICATION

(11) **EP 3 016 654 B2**
(45) Date of publication and mention of the opposition decision: **12.11.2025**
(45) Mention of the grant of the patent: 05.09.2018
(21) Application number: 13747521.6
(22) Date of filing: 01.07.2013
(51) Int. Cl.: A61K 31/428, A61K 31/575, A61K 9/00, A61P 25/00, A61P 25/16, A61P 25/28

(54) **TAUROURSODEOXYCHOLIC ACID (TUDCA) FOR USE IN THE TREATMENT OF NEURODEGENERATIVE DISORDERS**
TAUROURSODESOXYCHOLINSÄURE ZUR VERWENDUNG BEI DER BEHANDLUNG VON NEURODEGENERATIVEN ERKRANKUNGEN
ACIDE TAUROURSODÉSOXYCHOLIQUE (TUDCA) À UTILISER DANS LE TRAITEMENT DE TROUBLES NEURODÉGÉNÉRATIFS

(43) Date of publication of application: 11.05.2016
(73) Proprietor: Bruschettini S.r.l., 16147 Genova (IT)
(72) Inventor: RINALDI, Gilberto, I-16147 Genova (IT)
(74) Representative: Dragotti & Associati S.P.A.
(86) International application number: PCT/IB2013/055383
(87) International publication number: WO 2015/001379

(56) References cited:
- EP-A1- 2 422 787
- WO-A1-2006/086452
- WO-A2-2004/096123
- WO-A2-2006/050165
- CLINICAL TRIALS.GOV: "Efficacy and Tolerability of Tauroursodeoxycholic Acid in Amyotrophic Lateral Sclerosis (TUDCA-ALS)", 23 March 2012 (2012-03-23), XP002714173, Retrieved from the Internet <URL:http://www.clinicaltrials.gov/ct2/show/NCT00877604?term=tudca&rank=1> [retrieved on 20131002]
- K. TAUB: "TUDCA: Tauroursodeoxycholic Acid", 14 November 2004 (2004-11-14), XP002714174, Retrieved from the Internet <URL:http://www.stanford.edu/group/hopes/cgi-bin/wordpress/2010/06/tudca-tauroursodeoxycholic-acid/> [retrieved on 20131002]
- KEENE C D ET AL: "TAUROURSODEOXYCHOLIC ACID, A BILE ACID, IS NEUROPROTECTIVE IN A TRANSGENIC ANIMAL MODEL OF HUNTINGTON'S DISEASE", PROCEEDINGS OF THE NATIONAL ACADEMY OF SCIENCES - PNAS, NATIONAL ACADEMY OF SCIENCES, US, vol. 99, no. 16, 6 August 2002 (2002-08-06), pages 10671 - 10676, XP008038001, ISSN: 0027-8424, DOI: 10.1073/PNAS.162362299
- DUAN W-M ET AL: "TAUROURSODEOXYCHOLIC ACID IMPROVES THE SURVIVAL AND FUNCTION OF NIGRAL TRANSPLANTS IN A RAT MODEL OF PARKINSON'S DISEASE", CELL TRANSPLANTATION, COGNIZANT COMMUNICATION CORP, US, vol. 11, no. 3, 1 January 2002 (2002-01-01), pages 195 - 205, XP009064566, ISSN: 0963-6897

## Description

The object of the present invention is the use of TUDCA or a pharmaceutically acceptable salt thereof in the treatment of ALS. The recipient of such a treatment is a human.

### STATE OF THE ART

Amyotrophic lateral sclerosis (ALS) is a rapidly progressive and fatal neurodegenerative condition characterized by loss of upper and lower motor neurons of the central nervous system (1). Degeneration of lower motor neurons in the anterior horns of spinal cord and brainstem leads to progressive muscular atrophy and eventually to death within a few years due to respiratory insufficiency. A number of complex biochemical and regulatory pathways are likely involved in the pathogenesis of ALS (2). These different pathways interact with each other, eventually leading to controlled cell death or apoptosis, which is thought to be a common promoting factor for many neurodegenerative diseases, including ALS (3,4). In addition, recent evidence suggests a role for mitochondrial dysfunction in the pathogenesis of this invariably and rapidly fatal disease (5,6).

The development of a disease modifying therapy that may reverse progression of disability is a highest priority in ALS research. Thus far, only riluzole, a drug inhibiting glutamate release, has shown a modest disease modifying efficacy, as it prolonged survival by three months after 18 months of treatment, although with little if any effect on functional deterioration (7). Unfortunately, this approach has not proven satisfactory in stopping or significantly slowing the degenerative process of ALS (8,9).

Tauroursodeoxycholic acid (TUDCA) is a hydrophilic bile acid that is normally produced endogenously in humans at very low levels in the liver, by conjugation of taurine to ursodeoxycholic acid. This substance is commonly used as a bile acid replacement therapy for treatment of several chronic cholestatic liver diseases, for gallstone dissolution and as a gallstone formation preventative (10). TUDCA increases the cholesterol solubilizing activity of bile and in addition possesses many ancillary features, including the inhibition of mitochondrial-associated apoptosis through different mechanisms (11). TUDCA is also known for having a cytoprotective and anti-apoptotic action (12;13). TUDCA is a potent neuroprotective drug in transgenic animal model of Huntington disease and in stroke animal models (20;21). TUDCA improved the survival and function of nigral transplants in a rat model of Parkinson disease (22). More recently, it has been shown that TUDCA protected from secondary damage after spinal cord injury in rats (23). The therapeutic effect of ursodesossicolic acid may be ascribed to the successful delivery of TUDCA to the central nervous system (24) and its multiple cytoprotective actions.

In a recent publication an oral soluble ursodesossicolic acid formula was tested for three months in ALS patients with a cross-over, randomized, placebo-controlled design (19). A non-significant 34% relative slowing of the deterioration rate was observed in the treatment group compared to placebo.

There is therefore the need for an improved treatment that would stop or significantly slow the process of neurodegeneration and that would be well tolerated by the patients.

It has been now surprisingly found that the administration of TUDCA to patients refractory to traditional treatments results particularly efficient for treating neurodegenerative disorders, in particular for treating ALS, since it significantly slows the progress of the disease, thus improving the quality of life of the patients.

The object of the present invention is therefore the use of TUDCA or a pharmaceutically acceptable salt thereof in the treatment of ALS in a human, wherein it is administered for at least 30 weeks.

As used herein, the term "treatment of a neurodegenerative disorder" means stopping or significantly slowing the progress of such a neurodegenerative disorder.

According to the invention, TUDCA or a pharmaceutically acceptable salt thereof is administered to a human. Suitable routes of administration for TUDCA or a pharmaceutically acceptable salt thereof include oral route or parenteral route, preferably oral route. According to another embodiment, TUDCA or a pharmaceutically acceptable salt thereof is administered to patients refractory to traditional treatments, such as an ALS treatment based on riluzole. According to another embodiment, TUDCA or a pharmaceutically acceptable salt thereof is administered after a traditional treatment, such as an ALS treatment based on riluzole.

According to another embodiment, TUDCA or a pharmaceutically acceptable salt thereof is administered in combination with a traditional treatment, such as an ALS treatment based on riluzole (either with or without vitamin E).

According to another embodiment, TUDCA or a pharmaceutically acceptable salt thereof is administered at a dose of at least 1.5 g per day, preferably at a dose of about 2 g per day.

According to another embodiment, TUDCA or a pharmaceutically acceptable salt thereof is administered at least twice a day, more preferably at a dose of 1 g twice a day.

According to another embodiment, TUDCA is administered for at least 54 weeks.

A further object of the present invention is a pharmaceutical composition comprising TUDCA or a pharmaceutically acceptable salt thereof and at least one physiologically acceptable excipient for use in the treatment of ALS. According to an embodiment, the pharmaceutical composition of the present invention is administered as a combined preparation with riluzole for simultaneous, separate or sequential use.

### DESCRIPTION OF THE FIGURES

Figure 1. Study flowchart and patients disposition
Figure 2. Baseline-adjusted absolute values (left panel) and baseline-adjusted percentage changes (right panel) for the ALSFRS-R score at the end of the 54 weeks of treatment with placebo (open bars, n=17) or TUDCA (tauroursodeoxycholic acid, grey bars, n=17). Data are shown as averages and 95% confidence intervals for the full analysis set. The p-values refer to the statistical significance of the between-group differences.
Figure 3. Kaplan-Meier estimates of the probability of survival in the two group, placebo patients and TUDCA patients(P = 0.092; log-rank test).
Figure 4. Linear regression analysis: relationship between ALSFFRS-R mean scores and time (weeks) for TUDCA patients (black circle, slope= -0.388) and placebo patients (white circle slope= -0.262).

### EXPERIMENTAL SECTION

The present clinical study is aimed at evaluating the disease modifying action of TUDCA in ALS patients when added to riluzole.

### Study population

Eligible patients were aged 18-75 years and had clinically probable or definite ALS disease, as defined in the revised El Escorial criteria (14), with a disease duration of less than 12 months at study entry. Patients were required to have a forced vital capacity (FVC) equal to or greater than 75% of the predicted one, a steady treatment regimen with riluzole and vitamin E since at least 3 months and evidence of disease progression during the last 3 months. These inclusion criteria were aimed at recruiting patients who were not severely disabled, whose disease progression could be effectively monitored.

Patients were not eligible for the study were those previously submitted to a tracheostomy or a gall bladder resection, those with signs of motor or sensory nerve conduction blocks, with dementia, suffering from active peptic ulcer, or active malignancy. Pregnant women and breast-feeding mothers or women with child bearing potential, but not practicing an effective method of birth control were also excluded.

All eligible patients gave written informed consent to participate in this study, which was approved by the hospital ethics committee.

### Study design

This study is a phase II, multicenter (3 Italian centers), randomized (1:1), double-blind, placebo controlled, parallel group study. The study duration of study treatment was 54 weeks with either placebo or TUDCA (1 g b.i.d. *per os*), preceded by a 12-week run-in period with steady riluzole (50 mg b.i.d.) and vitamin E (300 mg q.d.), treatment. Randomization was independently performed by generating a randomized list that was kept blinded to the study investigators and the statisticians.

All patients were followed during the 12-week run-in observational period, during which they underwent two in-person visits, to assess concomitant medication, occurrence of adverse events, compliance to riluzole and vitamin E treatment and level of disability (ALSFRS-R). At the end of the run-in phase, the eligible patients entered a 54-week randomized treatment period, with in-person visits every 6 weeks. At randomization and at each follow-up visit blood samples for routine examinations were taken, and the following assessments were performed: physical function and progression of patients' disability using the ALSFRS-R scale (15), use of concomitant medications, occurrence of adverse events, and compliance to treatment. In addition, the following assessments were performed at randomization, at week 24 and at the end of treatment period: neurological physical examination, respiratory function (FVC), muscle strength (MRC Scale) (16), and the quality of life (SF-36 questionnaire) (17). Safety was assessed via adverse events monitoring (safety questionnaire and spontaneous reporting by the patients), vital signs and assessment of laboratory tests data for complete blood count, basic chemistry panel, liver function testing and creatine kinase (CK) carried out at each visit.

The primary study end-point was a between-treatment comparison of ALSFRS-R at study end. The study hypothesis was that treatment with TUDCA was superior to placebo in decreasing or delaying clinical disease activity in ALS patients. This was intended as a pilot study in order to obtain preliminary information on the possible therapeutic efficacy of TUDCA as add-on treatment in ALS patients also taking riluzole and vitamin E. Thus, no sample size estimation based on expected results was performed.

Analysis was performed with an intention-to-treat design on the full analysis set, including all randomized patients receiving at least one dose of the study medication and having at least one primary efficacy assessment after randomization, regardless of possible protocol violations. The last observation carried forward imputation method was used to replace missing values at study end for patients prematurely leaving the study.

Secondary efficacy outcomes included between-treatment comparison of: a) rate of responder patients, defined as all subjects showing an improvement of at least 15% in the ALSFRS-R slope during the treatment period as compared to baseline; b) FCV at the end of treatment; c) survival time during the follow-up; d) physical component summary (PCS) and mental component summary (MCS) scores of SF-36 at the end of treatment; e) MRC scores for right and left muscle groups at study end.

Homogeneity of clinical characteristics and efficacy variables at baseline between the two randomization groups was assessed by analysis of variance, in case of continuous variables, and by a Chi Square test in case of discrete variables. All efficacy endpoints were compared between the two randomization groups at study end by means of analysis of covariance, adjusting for baseline value and for center effect. Additionally, analysis of covariance with adjustment for baseline value was used to compare changes vs. baseline for some efficacy endpoints. Survival time was compared between treatments by a Kaplan-Meier survival analysis and a Cox proportional hazard model, using gender and age at ALS onset as covariates.

The level of statistical significance was kept at 0.05 throughout the whole study and all the results are reported with two-sided p-values. Data are shown as mean ±SD or as mean and 95% confidence interval (CI) (continuous variables) or as absolute (n) and relative frequency (categorical variables).

### Results

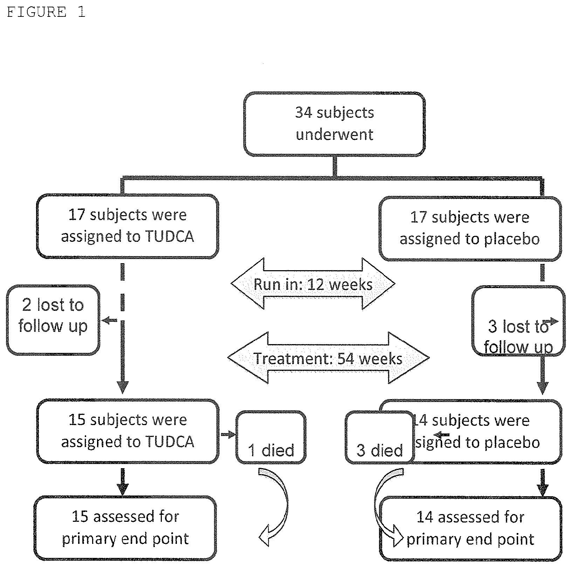

A total of 34 patients were randomized, 17 of which to placebo and 17 to TUDCA treatment (Figure 1). Of the 34 patients randomized to treatment, five were not included in the intention to treat (ITT) population because they drop out before visit 2. The full analysis set included 29 randomized patients, four of whom died during the study period (Figure 1). The patients' baseline characteristics were similar in both treatment groups (Table 1).

**Table 1.**

| | | Placebo (n=17) | TUDCA (n=17) | p-value |
|---|---|---|---|---|
| Age (years) | | 58.8±12.1 | 54.9±11.9 | 0.3505 |
| Gender | | | | |
| | *Males* | *10 (58.8)* | *11 (64.7)* | *0.7242* |
| | *Females* | 7 (41.2) | *6 (35.3)* | |
| ALSFRS-R Scale | | 38.4±6.4 | 38.7±4.9 | 0.8870 |
| FVC (%) | | 96.1±7.9 | 94.9±12.2 | 0.7358 |
| SF-36 | | | | |
| Questionnaire | | | | |
| | *PCS* | *38.0±7.0* | *39.3±9.9* | *0.695* |
| | *MCS* | *45.4±13.0* | *50.9±11.8* | *0.263* |
| MRC Scale | | | | |
| | *Right muscle group* | *56.9±7.6* | *58.2±3.9* | *0.626* |
| | *Left muscle group* | *54.9±9.0* | *55.6±8.9* | *0.851* |
| Abbreviations: ALSFRS-R: Amyotrophic Lateral Sclerosis Functional Rating Scale Revised; FVC: Forced Vital Capacity; SF-36: Short Form 36; PCS: Physical Component Summary; MCS: Mental Component Summary; MRC: Medical Research Council | | | | |

Demographic and clinical characteristics of the study population at the time of randomization. Data are shown as means±SD or as absolute value (n) and relative percentages (%) for the group of patients randomized to placebo and those randomized to tauroursodeoxycholic acid (TUDCA). The p-values refer to the statistical significance of the between-group differences.

Treatments were well tolerated in all patients, with no event adverse reported. Laboratory parameters were consistent with values obtained at the Baseline Visits. No remarkable mean changes in laboratory parameters were observed in either treatment group.

### Primary efficacy assessment.

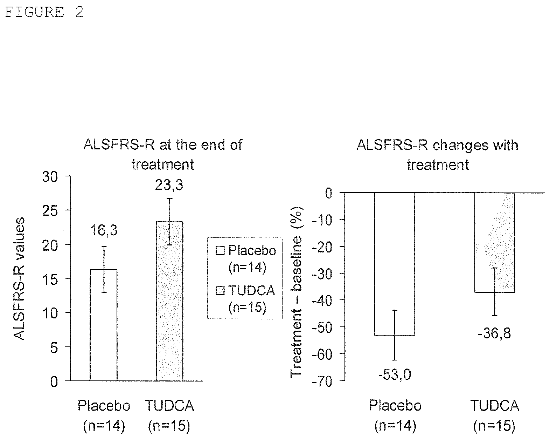

At study end the baseline-adjusted absolute ALSFRS-R score was significantly higher in TUDCA-treated than in placebo-treated patients [mean value and 95% CI: 23.3 (19.9 to 26.6) vs. 16.3 (12.9 to 19.7)] (p=0.007; Figure 2). Baseline ALSFRS-R progressively declined during the treatment period to the end of the study, but to a significantly less extent under TUDCA than under placebo [mean percentage reduction and 95% CI: 53.0 (62.3 to 43.7) vs. 36.8 (45.8 to 27.9)] (p=0.016).

### Secondary efficacy assessments.

The rate of responder patients was significantly higher under TUDCA (87%) than placebo (43%) (p=0.021). The other secondary outcomes did not significantly differ between treatment groups (Table 2).

**Table 2.**

| | | Placebo (baseline) | Placebo (54 weeks) | TUDCA (baseline) | TUDCA (54 weeks) | p-value |
|---|---|---|---|---|---|---|
| FVC (%) | | 96.1 (90.0-102.2) | 87.7 (80.9-95.3) | 94.9 (86.8-101.4) | 89.1 (81.4-96.7) | 0.778 |
| SF-36 | | | | | | |
| Questionnaire | | | | | | |
| | *PCS* | 38.0 (33.8-42.2) | 35.0 (30.4-39.6) | 39.3 (33.3-45.3) | 34.8 (30.2-39.4) | 0.951 |
| | *MCS* | 45.4 (37.5-53.2) | 42.3 (35.5-49.2) | 50.9 (43.8-58.1) | 49.0 (42.1-55.8) | 0.173 |
| MRC Scale | | | | | | |
| | *Right muscle group* | 56.91 (51.8-62.0) | 47.0 (35.6-58.5) | 58.2 (55.6-60.8) | 49.2 (44.9-53.4) | 0.695 |
| | *Left muscle group* | 54.9 (48.8-61.0) | 43.7 (32.9-54.6) | 55.6 (49.6-61.6) | 47.0 (41.6-52.4) | 0.553 |
| Abbreviations: | | | | | | |
| FVC: Forced Vital Capacity; SF-36: Short Form 36; PCS: Physical Component Summary; MCS: Mental Component Summary; MRC: Medical Research Council | | | | | | |

Secondary outcome measures, Data are shown as relative percentages (%) or baseline-adjusted mean values and 95% confidence intervals for the group of patients randomized to placebo and those randomized to tauroursodeoxycholic acid (TUDCA). The p-values refer to the statistical significance of the between-group differences.

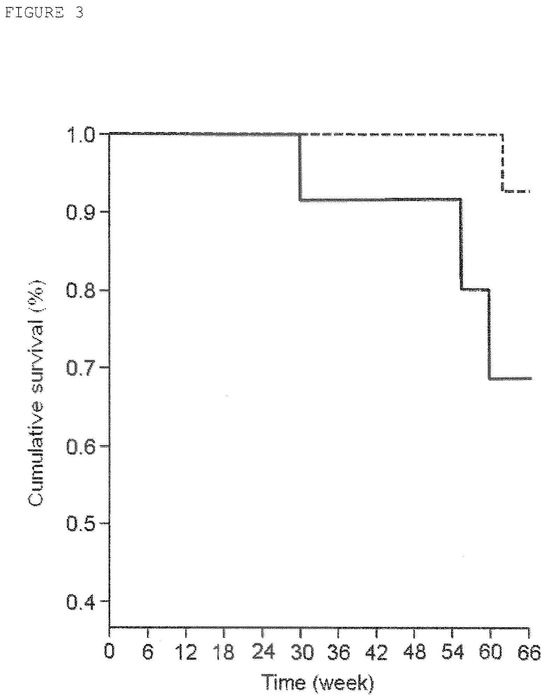

Cumulative incidence of death in the course of the observation period (median follow-up of 66 weeks) tended to be higher in the placebo group (3 deaths, 18% of patients) than in the TUDCA group (1 death, 6% of patients) (Figure 3). Accordingly, the average survival time was a bit longer under TUDCA treatment [mean value and 95% CI: 65.7 (65.2 to 66.3) vs. 61.1 (55.3 to 66.9) weeks with placebo].

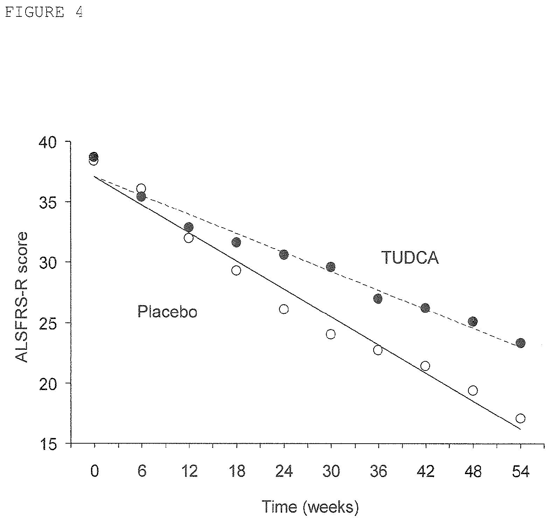

At the end of the study period (54 weeks) the ALSFRS-R mean score in the TUDCA group corresponded to the mean value reached by the placebo group at the week 36, i.e. 18 weeks earlier (Figure 4). The slopes of the two regression lines were different (-0.262 for the TUDCA group, -0.388 for the placebo group; p < 0.01).

### Discussion

This study shows that one year of treatment with TUDCA at the prescribed dose was associated with slower deterioration of function in ALS patients. This disease modifying effect is additional to that of riluzole, as both treatment groups were under riluzole and vitamin E treatment. At the end of the yearly study period (54 weeks) patients in the TUDCA group scored 23 on the ALSFRS-R, corresponding to the mean value scored by the placebo group at the week 36. This suggests that the one-year TUDCA treatment may slow ALS deterioration by 18 weeks. The observed trend of progression furthermore suggests that longer durations of treatment may produce further between-group divergence. In keeping with this, the number of "responder" patients was significantly higher in the TUDCA group.

The non-significant outcome on other secondary endpoints may depend on the small sample size of this pilot study. Notwithstanding, a lesser decline in lung function was observed in TUDCA-treated patients, indicating a possible positive impact of treatment on respiratory involvement. A progressively reduced FVC during the disease course is known to be associated with a poor patient's prognosis (18). In addition, TUDCA treatment was associated with a less pronounced deterioration in muscle strength and in quality of life. Finally, a trend to a lower mortality rate in the TUDCA group was seen. Whether these measures will prove significant in larger future studies is presently unknown.

The treatment was well tolerated by all patients. Except for the expected complications of ALS such as dyspnea and dysphagia, no adverse events that could possibly be attributed to the study drug or placebo were recorded; moreover the values of vital signs and laboratory values were stable during the study period. The results of this study are intriguing because they show that the disease modifying effect exerted by TUDCA are additional to those of riluzole, suggesting that more than one compound may be used to slow down disease progression in ALS. The TUDCA dose used in this trial (2 g per day) is higher than that usually recommended for treating hepatic diseases (500 to 750 mg per day). However, considering that it was well tolerated we cannot exclude that even higher oral doses or intravenous delivery of TUDCA could be used.

### REFERENCE LIST

(1) Ince PG, Lowe J, Shaw PJ. Amyotrophic lateral sclerosis: current issues in classification, pathogenesis and molecular pathology. Neuropathol Appl Neurobiol 1998 Apr;24(2):104-17.
(2) Rowland LP, Shneider NA. Amyotrophic lateral sclerosis. N Engl J Med 2001 May 31;344(22):1688-700.
(3) Mitchell JD, Borasio GD. Amyotrophic lateral sclerosis. Lancet 2007 Jun 16;369(9578):2031-41.
(4) Sathasivam S, Shaw PJ. Apoptosis in amyotrophic lateral sclerosis--what is the evidence? Lancet Neurol 2005 Aug;4(8):500-9.
(5) Caroppi P, Sinibaldi F, Fiorucci L, Santucci R. Apoptosis and human diseases: mitochondrion damage and lethal role of released cytochrome C as proapoptotic protein. Curr Med Chem 2009; 16(31) :4058-65.
(6) Rizzardini M, Lupi M, Bernasconi S, Mangolini A, Cantoni L. Mitochondrial dysfunction and death in motor neurons exposed to the glutathione-depleting agent ethacrynic acid. J Neurol Sci 2003 Mar 15;207(1-2):51-8.
(7) Lacomblez L, Bensimon G, Leigh PN, Guillet P, Meininger V. Dose-ranging study of riluzole in amyotrophic lateral sclerosis. Amyotrophic Lateral Sclerosis/Riluzole Study Group II. Lancet 1996 May 25; 347(9013) :1425-31.
(8) Leigh PN, Swash M, Iwasaki Y, Ludolph A, Meininger V, Miller RG, et al. Amyotrophic lateral sclerosis: a consensus viewpoint on designing and implementing a clinical trial. Amyotroph Lateral Scler Other Motor Neuron Disord 2004 Jun;5(2):84-98.
(9) Ludolph AC, Brettschneider J, Weishaupt JH. Amyotrophic lateral sclerosis. Curr Opin Neurol 2012 Oct;25(5):530-5.
(10) Hofmann AF. The continuing importance of bile acids in liver and intestinal disease. Arch Intern Med 1999 Dec 13;159(22):2647-58.
(11) Amaral JD, Xavier JM, Steer CJ, Rodrigues CM. The role of p53 in apoptosis. Discov Med 2010 Feb;9(45):145-52.
(12) Amaral JD, Viana RJ, Ramalho RM, Steer CJ, Rodrigues CM. Bile acids: regulation of apoptosis by ursodeoxycholic acid. J Lipid Res 2009 Sep;50(9):1721-34.
(13) Jain K. The Handbook of Neuroprotection. New York: Springer; 2011.
(14) Brooks BR, Miller RG, Swash M, Munsat TL. El Escorial revisited: revised criteria for the diagnosis of amyotrophic lateral sclerosis. Amyotroph Lateral Scler Other Motor Neuron Disord 2000 Dec;1(5):293-9.
(15) Cedarbaum JM, Stambler N, Malta E, Fuller C, Hilt D, Thurmond B, et al. The ALSFRS-R: a revised ALS functional rating scale that incorporates assessments of respiratory function. BDNF ALS Study Group (Phase III). J Neurol Sci 1999 Oct 31;169(1-2):13-21.
(16) Medical Research Council. Aids to the examination of the peripheral nervous system, Memorandum no. 45. Her Majesty's Stationery Office ed. London: 1981.
(17) Jenkinson C, Hobart J, Chandola T, Fitzpatrick R, Peto V, Swash M. Use of the short form health survey (SF-36) in patients with amyotrophic lateral sclerosis: tests of data quality, score reliability, response rate and scaling assumptions. J Neurol 2002 Feb;249(2):178-83.
(18) Czaplinski A, Yen AA, Appel SH. Forced vital capacity (FVC) as an indicator of survival and disease progression in an ALS clinic population. J Neurol Neurosurg Psychiatry 2006 Mar;77(3):390-2.
(19) Min JH, Hong YH, Sung JJ, Kim SM, Lee JB, Lee KW. Oral solubilized ursodeoxycholic acid therapy in amyotrophic lateral sclerosis: a randomized cross-over trial. J Korean Med Sci 2012 Feb;27(2):200-6.
(20) Keene CD, Rodrigues CM, Eich T, Chhabra MS, Steer CJ, Low WC. Tauroursodeoxycholic acid, a bile acid, is neuroprotective in a transgenic animal model of Huntington's disease. Proc Natl Acad Sci U S A 2002 Aug 6;99(16):10671-6.
(21) Rodrigues CM, Sola S, Nan Z, Castro RE, Ribeiro PS, Low WC, et al. Tauroursodeoxycholic acid reduces apoptosis and protects against neurological injury after acute hemorrhagic stroke in rats. Proc Natl Acad Sci U S A 2003 May 13; 100(10):6087-92.
(22) Duan WM, Rodrigues CM, Zhao LR, Steer CJ, Low WC. Tauroursodeoxycholic acid improves the survival and function of nigral transplants in a rat model of Parkinson's disease. Cell Transplant 2002;11(3) :195-205.
(23) Macedo B, Batista AR, Ferreira N, Almeida MR, Saraiva MJ. Anti-apoptotic treatment reduces transthyretin deposition in a transgenic mouse model of Familial Amyloidotic Polyneuropathy. Biochim Biophys Acta 2008 Sep;1782(9):517-22.
(24) Parry GJ, Rodrigues CM, Aranha MM, Hilbert SJ, Davey C, Kelkar P, et al. Safety, tolerability, and cerebrospinal fluid penetration of ursodeoxycholic Acid in patients with amyotrophic lateral sclerosis. Clin Neuropharmacol 2010 Jan;33(1):17-21.

## Claims

1. Tauroursodeoxycholic acid or a pharmaceutically acceptable salt thereof for use in the treatment of amyotrophic lateral sclerosis in a human, **characterized in that** it is administered for at least 30 weeks.

2. Tauroursodeoxycholic acid or a pharmaceutically acceptable salt thereof for use according to claim 1, **characterized in that** it is administered by oral or parenteral route, preferably by oral route.

3. Tauroursodeoxycholic acid or a pharmaceutically acceptable salt thereof for use according to any one of claims 1-2, **characterized in that** said tauroursodeoxycholic acid is administered at a dose of at least 1.5 g per day, preferably of about 2 g per day.

4. Tauroursodeoxycholic acid or a pharmaceutically acceptable salt thereof for use according to any one of claims 1-3, **characterized in that** said tauroursodeoxycholic acid is administered at least twice a day, preferably at a dose of 1 g twice a day.

5. Tauroursodeoxycholic acid or a pharmaceutically acceptable salt thereof for use according to any one of claims 1-4, **characterized in that** it is administered for at least 54 weeks.

6. Tauroursodeoxycholic acid or a pharmaceutically acceptable salt thereof for use according to any one of claims 1-5, **characterized in that** it is administered to patients refractory to traditional amyotrophic lateral sclerosis treatments.

7. Tauroursodeoxycholic acid or a pharmaceutically acceptable salt thereof for use according to any one of claims 1-6, **characterized in that** it is administered after a treatment based on riluzole.

8. Tauroursodeoxycholic acid or a pharmaceutically acceptable salt thereof for use according to any one of claims 1-6, **characterized in that** it is administered in combination with riluzole.

9. Tauroursodeoxycholic acid or a pharmaceutically acceptable salt thereof for use according to claim 8, **characterized in that** it administered simultaneously, separately or sequentially with respect to riluzole.

## Patentansprüche

1. Tauroursodeoxycholsäure oder ein pharmazeutisch annehmbares Salz davon zur Verwendung bei der Behandlung vom amyotropher Lateralsklerose in einem Menschen, **dadurch gekennzeichnet, dass** sie für mindestens 30 Wochen verabreicht wird.

2. Tauroursodeoxycholsäure oder ein pharmazeutisch annehmbares Salz davon zur Verwendung nach Anspruche 1, **dadurch gekennzeichnet, dass** es über die orale oder parenterale Route verabreicht wird, vorzugsweise über die orale Route.

3. Tauroursodeoxycholsäure oder ein pharmazeutisch annehmbares Salz davon zur Verwendung nach einem der Ansprüche 1-2, **dadurch gekennzeichnet, dass** die Tauroursodeoxycholsäure in einer Dosis von mindestens 1,5 g pro Tag, vorzugsweise etwa 2 g pro Tag verabreicht wird.

4. Tauroursodeoxycholsäure oder ein pharmazeutisch annehmbares Salz davon zur Verwendung nach einem der Ansprüche 1-3, **dadurch gekennzeichnet, dass** die Tauroursodeoxycholsäure mindestens zweimal pro Tag, vorzugsweise in einer Dosis von 1 g zweimal pro Tag verabreicht wird.

5. Tauroursodeoxycholsäure oder ein pharmazeutisch annehmbares Salz davon zur Verwendung nach einem der Ansprüche 1-4, **dadurch gekennzeichnet, dass** sie für mindestens 54 Wochen verabreicht wird.

6. Tauroursodeoxycholsäure oder ein pharmazeutisch annehmbares Salz davon zur Verwendung nach einem der Ansprüche 1-5, **dadurch gekennzeichnet, dass** sie Patienten verabreicht wird, die herkömmlichen Behandlungen amyotropher Lateralsklerose widerstehen.

7. Tauroursodeoxycholsäure oder ein pharmazeutisch annehmbares Salz davon zur Verwendung nach einem der Ansprüche 1-6, **dadurch gekennzeichnet, dass** sie nach einer Behandlung basierend auf Riluzol verabreicht wird.

8. Tauroursodeoxycholsäure oder ein pharmazeutisch annehmbares Salz davon zur Verwendung nach einem der Ansprüche 1-6, **dadurch gekennzeichnet, dass** sie im Kombination mit Riluzol verabreicht wird.

9. Tauroursodeoxycholsäure oder ein pharmazeutisch annehmbares Salz davon zur Verwendung nach Anspruch 8, **dadurch gekennzeichnet, dass** sie gleichzeitig, getrennt oder nacheinander in Bezug zu Riluzol verabreicht wird.

## Revendications

1. Acide tauroursodésoxycholique ou sel pharmaceutiquement acceptable de celui-ci destiné à être utilisé dans le traitement de la sclérose latérale amyotrophique chez l'humain, **caractérisé en ce qu'**il est administré pendant au moins 30 semaines.

2. Acide tauroursodésoxycholique ou sel pharmaceutiquement acceptable de celui-ci destiné à être utilisé selon la revendication 1, **caractérisé en ce qu'**il est administré par voie orale ou parentérale, de préférence par voie orale.

3. Acide tauroursodésoxycholique ou sel pharmaceutiquement acceptable de celui-ci destiné à être utilisé selon l'une quelconque des revendications 1 à 2, **caractérisé en ce que** ledit acide tauroursodésoxycholique est administré à une dose d'au moins 1,5 g par jour, de préférence d'environ 2 g par jour.

4. Acide tauroursodésoxycholique ou sel pharmaceutiquement acceptable de celui-ci destiné à être utilisé selon l'une quelconque des revendications 1 à 3, **caractérisé en ce que** ledit acide tauroursodésoxycholique est administré au moins deux fois par jour, de préférence à une dose de 1 g deux fois par jour.

5. Acide tauroursodésoxycholique ou sel pharmaceutiquement acceptable de celui-ci destiné à être utilisé selon l'une quelconque des revendications 1 à 4, **caractérisé en ce qu'**il est administré pendant au moins 54 semaines.

6. Acide tauroursodésoxycholique ou sel pharmaceutiquement acceptable de celui-ci destiné à être utilisé selon l'une quelconque des revendications 1 à 5, **caractérisé en ce qu'**il est administré à des patients réfractaires aux traitements traditionnels de la sclérose latérale amyotrophique.

7. Acide tauroursodésoxycholique ou sel pharmaceutiquement acceptable de celui-ci destiné à être utilisé selon l'une quelconque des revendications 1 à 6, **caractérisé en ce qu'**il est administré après un traitement à base de riluzole.

8. Acide tauroursodésoxycholique ou sel pharmaceutiquement acceptable de celui-ci destiné à être utilisé selon l'une quelconque des revendications 1 à 6, **caractérisé en ce qu'**il est administré en combinaison avec du riluzole.

9. Acide tauroursodésoxycholique ou sel pharmaceutiquement acceptable de celui-ci destiné à être utilisé selon la revendication 8, **caractérisé en ce qu'**il est administré simultanément, séparément ou séquentiellement par rapport au riluzole.
